# EUROPÄISCHE PATENTSCHRIFT

(11) **EP 1 165 509 B1**
(45) Veröffentlichungstag und Bekanntmachung des Hinweises auf die Patenterteilung: **06.10.2004**
(21) Anmeldenummer: 00918809.5
(22) Anmeldetag: 17.03.2000
(51) Int. Cl.: C07D 207/22, C07D 211/78

(54) **VERFAHREN ZUR HERSTELLUNG VON N-ACYL-3,4-DEHYDROPROLIN UND N-ACYL-3,4-DEHYDROPIPERIDIN-2-CARBONSÄURE DERIVATEN**
METHOD OF PRODUCING 3,4-DIHYDROPROLINES AND 3,4-DEHYDROPIPERIDINES
PROCEDE DE PREPARATION DE DERIVES DE N-ACYLE-3,4-DESHYDROPROLINE ET D'ACIDE N-ACYLE-3,4 - DESHYDROPIPERIDINE-2-CARBOXYLIQUE

(30) Priorität: 26.03.1999 DE 19913699
(43) Veröffentlichungstag der Anmeldung: 02.01.2002
(73) Patentinhaber: Abbott GmbH & Co. KG, 65205 Wiesbaden (DE)
(72) Erfinder: SCHÄFER, Bernd, D-76889 Dierbach (DE); HELMCHEN, Günter, D-69115 Heidelberg (DE); KAZMAIER, Uli, D-69168 Wiesloch (DE); SCHLEICH, Simone, D-69124 Heidelberg (DE); STAHR, Helmut, D-79539 Lörrach (DE); WOLFART, Volker, CH-5036 Oberentfelden (CH)
(74) Vertreter: Grünecker, Kinkeldey, Stockmair & Schwanhäusser Anwaltssozietät
(86) Internationale Anmeldenummer: PCT/EP2000/002377
(87) Internationale Veröffentlichungsnummer: WO 2000/058284

(56) Entgegenhaltungen:
- WO-A-98/04523
- WO-A-98/55456
- US-A- 4 291 040
- AMINO Y. ET AL.: "Synthesis of proline and 2-piperidinecarboxylic acid via cobalt-catalyzed isomerization-carbonylation of N-acyl unsaturated cyclic amines" BULLETIN OF THE CHEMICAL SOCIETY OF JAPAN, JP, JAPAN PUBLICATIONS TRADING CO. TOKYO, Bd. 64, Nr. 2, 1991, Seiten 620-623, XP000909953 ISSN: 0009-2673 in der Anmeldung erwähnt
- DONOHOE T.J. ET AL.: "Reduction of electron-deficient pyrroles using group I and II metals in ammonia" JOURNAL OF THE CHEMICAL SOCIETY, PERKIN TRANSACTIONS 1, GB, CHEMICAL SOCIETY. LETCHWORTH, Nr. 4, 21. Februar 1998 (1998-02-21), Seiten 667-676, XP002080379 ISSN: 0300-922X

## Beschreibung

Die vorliegende Erfindung betrifft ein Verfahren zur Herstellung von 3,4-Dehydroprolinen und 3,4-Dehydropiperidinen. Insbesondere betrifft die Erfindung ein Verfahren zur Herstellung von Verbindungen der allgemeinen Formel I 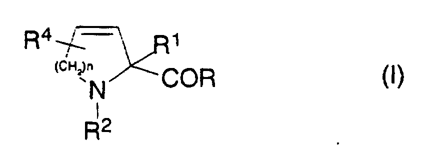
- R: Wasserstoff, C₁-C₆-Alkoxy, -NHC₁-C₆-Alkyl, -N(C₁-C₆-Alkyl)₂, OH, NH₂
- R¹: Wasserstoff, C₁-C₆-Alkyl, Me₃Si, C₁-C₆-Alkyl-S
- R²: Boc, C₁-C₆-Acyl, Mesyl, Benzolsulfonyl, Tosyl, Trifluoracetyl, A1-A3-Peptid
- n: 1, 2
- R⁴: H, C₁-C₆-Alkylrest.

3,4-Dehydroproline stellt man ausgehend von 4-Hydroxyprolin via Tschugaeff-Reaktion her (P. Grogg, Angew. Chem. 92 (1980) 761). Neben den vergleichsweise schlechten Ausbeuten (64 %) erfordert diese Methode den Umgang mit hochtoxischen Verbindungen wie Schwefelkohlenstoff, Methyliodid und Methylmercaptan. Die pyrolytische Zersetzung bei 180 bis 190°C und 12 Torr erfordert einen erhöhten technischen Aufwand.

Durch Thermolyse kann man anstelle der Xanthogenate auch entsprechende Iodide, Sulfoxide oder Selenoxide umsetzen (J.-R. Dormoy, Synthesis (1982) 752). Die grundlegenden Probleme hinsichtlich Toxizität und technischem Aufwand werden hierdurch nicht gelöst.

Achirale Synthesen gehen üblicherweise von Pyrrolcarbonsäure aus, die mit Phosphoniumiodid/Iodwasserstoff reduziert wird (J.W. Scott, Synth. Commun. 10 (1980) 529). Anschließend trennt man das Racemat mittels Kristallisation mit chiralen Aminen (S.S. Kerwar, J. Bio. Chem. 251 (1976) 503; US 4066658) oder Weinsäure (A. Corbella, Chem. Ind. (1969) 583). Der Nachteil dieser Synthese ist der Umgang mit dem hochtoxischen Phosphan und eine maximale Ausbeute von 50 % bei der Racematspaltung.

In WO 98/04523 ist die Eliminierung von Sulfonsäureestern des Hydroxyprolinesters und die anschließende enzymatische Racematspaltung beschrieben.

Die Birch-Reduktion von Pyrrol-Derivaten war bis vor kurzem unbekannt. T.J. Donohoe beschrieb in J. Org. Chem. 61 (1996) 7664 erstmals die achirale Birch-Reduktion von Pyrrol-2-carbonsäure-Derivaten. Diese konnten wie oben beschrieben bisher nur durch klassische oder enzymatische Racematspaltung in die Enantiomere getrennt werden.

In WO 98/55456 ist die diastereoselektive Birch-Reduktion chiraler Pyrrol-2-carbonsäureester und -amide beschrieben.

Die Synthese von 3,4-Dehydropiperidin-2-carbonsäurederivaten ist in D'Ambra, Bell, J. Org. Chem. 54 (1989) 5632, und in Krogsgaard-Larsen, J. Labbeled Compd 19 (1982) 689, beschrieben. Beide Synthesen setzen den Umgang mit extrem toxischen Chemikalien (Isocyanate, Nitrosamine) voraus und liefern das gewünschte Produkt nur in schlechten Ausbeuten.

Der Erfindung lag also die Aufgabe zugrunde, 3,4-Dehydroproline und 3,4-Dehydropiperidine der allgemeinen Formel 1 mittels einer einfachen Reaktionssequenz herzustellen.

Die Herstellung von 3-Pyrrolin z.B. via Metathese ist in der neueren Literatur umfassend dokumentiert (Grubbs, J. Org. Chem. 62 (1997) 7310; Pandit, Tetrahedron Lett. 37 (1996) 547; Grubbs, J. Am. Chem. Soc. 115 (1993) 9856; Moreno-Manas, Tetrahedron 54 (1998) 14869.).

Bekannt sind Alkylierungen des 3-Pyrrolins in 2-Position, z.B. Meyers, J. Am. Chem. Soc 107 (1985) 7974; Macdonald, J. Org. Chem. 45 (1980) 193; Francke, Liebigs Ann. (1995) 193 und die Hydroformylierung, die zu Derivaten des Prolins führt (Izawa, Bull. Chem. Soc. Jpn 64 (1991) 620.).

Carboxylierungen des Pyrrolidins sind sehr gut bekannt, z.B. Beak, J. Am. Chem. Soc. 116 (1994) 3231. Allerdings ist die Lehre aus Colegate, Austal. J. Chem. 37 (1984) 1503, daß bei einer analogen Deprotonierung von Methoxycarbonyl-3-pyrrolin dieses eine unerwünschte intermolekulare Reaktion eingeht und mit einer Ausbeute von 65 % N-Methoxycarbonyl-3-pyrrolin-2-carbonsäure-1-(3-pyrrolinid) ergibt.

Überraschenderweise wurde gefunden, daß sich Pyrroline und 3,4-Dehydropiperidine der allgemeinen Formel II 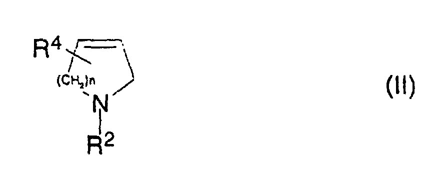 wobei
- R²: Boc, C₁-C₆-Acyl, Mesyl, Benzolsulfonyl, Tosyl, Trifluoracetyl, A1-A3-Peptid
- R⁴: H, C₁-C₆-Alkylrest
- n: 1 oder 2
bedeutet,
in Gegenwart eines Carboxylierungsreagenz oder Carbonylierungsreagenz der allgemeinen Formel III 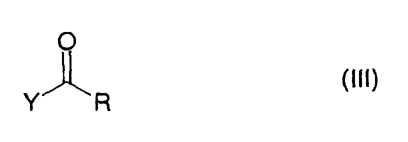 wobei
- R: Wasserstoff, C₁-C₆-Alkoxy, -NHC₁-C₆-Alkyl, -N(C₁-C₆-Alkyl)₂, OH, NH₂
- Y: Cl, C₁-C₆-Alkoxy, -NHC₁-C₆-Alkyl, -N(C₁-C₆-Alkyl)₂, N(C₁-C₆-Alkyl)OC₁-C₆, wobei R nicht OH ist,
bedeutet,
oder für R = OH in Formel I mit CO₂ umgesetzt wird, zusammen mit einer starken Base, bevorzugt einem Alkalimetallamid und hydrolysiert oder mit einem Reagenz der allgemeinen Formel IV

R³― X (IV)

wobei
- X: Cl, Br, I, MesO, TosO, Triflat
- R³: Wasserstoff, C₁-C₆-Alkyl, Me₃Si, C₁-C₆-Alkyl-S oder NH₄ oder
- R³-X: gleich (C₁-C₆-Alkyl-S)₂
bedeutet,
zu den gewünschten Dehydroprolinen und Dehydropiperidinen in guten Ausbeuten umsetzen läßt.
Bevorzugte Alkalimetallamide sind Lithium- und Natriumamide der allgemeinen Formel V

MNR⁵R⁶ (V)

mit
- M: Na, Li,
- R⁵: H, C₁-C₆-Alkyl
- R⁶: H, C₁-C₆-Alkyl.

Bevorzugte Bedeutung der Formel III ist Di-C₁-C₆-Alkylcarbonat, insbesondere Dimethylcarbonat und Diethylcarbonat.

Unter A1-A3-Peptid wird ein bis zu 3 Aminosäuren enthaltender Rest verstanden, wobei unter Aminosäuren natürliche (proteinogene) und nicht natürliche (nicht-proteinogene) Aminosäuren verstanden werden. Das A1-A3-Peptid kann derivatisiert oder mit üblichen Schutzgruppen geschützt sein. Unter A1-A3-Peptid werden auch teil- oder voll peptidomimetische Strukturen verstanden.

Insbesondere werden unter A1, A2 und A3 folgende Aminosäuren verstanden:
t-Butylglycin, t-Butylalanin, Adamantylglycin, Adamantylalanin, natürliche Aminosäuren, deren D-Enantiomere, Cyclopropylglycin, Cycloheptylglycin, Cycloheptylalanin, Cyclobutylglycin, Cyclopentylglycin, Cyclohexylglycin, Cyclopropylalanin, Cyclobutylalanin, Cyclopentylalanin, Cyclohexylalanin, sämtliche Isomere des Furanylglycin, Furanylalanin, Naphthylglycin, Naphthylalanin, Thiophenylglycin, Thiophenylalanin, Isochinolinglycin, Isochinolinalanin, Chinolinglycin, Chinolinalanin, Pyrrolylglycin, Pyrrolylalanin, Imidazolylglycin, Imidazolylalanin, 3,4-Dehydroprolin.

Die Umsetzung erfolgt in unter den Reaktionsbedingungen inerten Lösungsmitteln. Bevorzugte Lösungsmittel sind C₂-C₈-Kohlenwasserstoffe, insbesondere Hexane, THF und C₁-C₆-Ether, C₁-C₆-Ether/DMPU-Gemische, Dioxan sowie Gemische der aufgeführten Lösungsmittel.

Die Reaktion führt man im allgemeinen im Temperaturbereich von -100 bis +100°C und einem Druckbereich von 1 bis 200 bar durch. Bevorzugt ist der Temperaturbereich von -20 bis +20°C.

In der Regel bricht man die Reaktion in üblicher Weise ab, wenn keine Pyrrolin- bzw. Dehydropiperidin-Derivate im Reaktionsgemisch mehr nachgewiesen werden können (z.B. mittels GC, HPLC, DC).

Die Aufarbeitung auf das Verfahrensprodukt hin erfolgt dann in der Regel nach herkömmlichen Verfahren, wie Destillation, Filtration, Zentrifugation oder Extraktion.

Das erfindungsgemäße Verfahren ist diskontinuierlich, z.B. in einem Rührreaktor, durchzuführen. Die einfache Durchführbarkeit bietet jedoch den Vorteil, daß man auf kontinuierliche Arbeitsweise, beispielsweise unter Verwendung eines Reaktionsrohres oder einer Rührreaktorkaskade, umstellen kann.

Die erhaltenen Rohprodukte können gewünschtenfalls weiter gereinigt werden, z.B. durch Kristallisation, Extraktion oder Chromatographie.

Die nach dem erfindungsgemäßen Verfahren auf einfache Weise herzustellenden 3,4-Dehydroproline und 3,4-Dehydropiperidine der allgemeinen Formel I sind wertvolle Zwischenprodukte zur Synthese von Farbstoffen, Pflanzenschutzmitteln oder Arzneimitteln, insbesondere Thrombininhibitoren, wie z.B. in den Druckschriften WO 94/29336, WO 95/35309, WO 96/17860, WO 96/24609, WO 96/25426, WO 98/06741 beschrieben.

### Beispiele

### Beispiel 1

### N-^{t}Butoxycarbonyl-Δ³-dehydroprolinmethylester

4,1 ml (48,7 mmol) Dimethylcarbonat wurden mit 8,2 g (48,5 mmol) N-^{t}Butoxycarbonyl-Δ³-Pyrrolin in 40 ml THF gelöst und auf ca. -5°C abgekühlt. Dazu wurden 54 ml LDA Lösung (2 molar in Heptan, THF, Ethylbenzol) so zugetropft, daß die Innentemperatur nicht über 4°C stieg. Die Zugabe war nach etwa 20 min beendet. Die Reaktionsmischung hatte eine rot-braune Färbung angenommen. Nach 10 minütigem Nachrühren bei 0°C wurde das Reaktionsgemisch mit 150 ml n-Pentan verdünnt und auf 200 ml 1 N HCl gegeben. Nach Trennung der Phasen wurde 3 mal mit 50 ml n-Pentan extrahiert und die vereinigten organischen Phasen nacheinander je 2 mal mit 0,01 N HCl, gesättigter NaHCO₃-Lösung und gesättigter NaCl-Lösung gewaschen. Abschließend wurde über MgSO₄ getrocknet und die flüchtigen Bestandteile am Rotationsverdampfer entfernt. Das gelb-braune Rohprodukt wurde bei 120°C bis 150°C/0,4 Torr (53,3 Pa) destilliert. Es wurde ein schwach gelb gefärbtes Öl erhalten, das nach Einbringen von Impfkristallen auskristallisiert. Zur abschließenden Reinigung wurde aus n-Pentan umkristallisiert. Ausbeute: 9,06 g; 82 % d.Th.; farblose, klare Kristalle.

### Beispiel 2

### N-^{t}Butoxycarbonyl-2-methyl-Δ³-dehydroprolinmethylester

Der N-^{t}Butoxycarbonyl-Δ³-dehydroprolinmethylester wird wie unter Beispiel 1 beschrieben hergestellt (Ansatzgröße 1,60 mmol = 271 mg N-^{t}Butoxycarbonyl-Δ³-Pyrrolin. Nachdem die Zugabe des Lithiumdiisopropylamids beendet war, wurde für 5 min bei 0°C nachgerührt. Anschließend wurde bei dieser Temperatur 0,1 ml (1,61 mmol) Methyliodid zugegeben. Danach wurde für 10 min bei 0°C gerührt und im Anschluß wie unter Beispiel 1 aufgearbeitet. Es wurden 320 mg (83 % d.Th.) eines braunen Öls erhalten, das nach gaschromatographischer Analyse aus 24 % des N-^{t}Butoxycarbonyl-Δ³-dehydroprolinmethylesters und 75 % des gewünschten Produkts zusammengesetzt ist.

### Beispiel 3

### N-Ethoxycarbonyl-Δ³-piperidin-2-carbonsäuremethylester

Eine Lösung von 228 mg (1,47 mmol) N-Ethoxycarbonyl-Δ³-piperidin und 0,14 ml (1,66 mmol) Dimethylcarbonat in 3 ml THF wurde auf 0°C gekühlt und anschließend tropfenweise mit 1,5 ml LDA Lösung (2 molar in Heptan, THF, Ethylbenzol) versetzt. Diese Reaktionsmischung wurde für 10 min bei 0°C gerührt und anschließend auf eine Mischung von 5 ml 1N HCl und 10 ml tert.-Butylmethylester gegeben . Nach Trennung der Phasen wurde 3 mal mit 10 ml n-Pentan extrahiert und die vereinigten organischen Phasen nacheinander je 2 mal mit 0,01 N HCl, gesättigter NaHCO₃-Lösung und gesättigter NaCl-Lösung gewaschen. Danach wurde über MgSO₄ getrocknet und die flüchtigen Bestandteile am Rotationsverdampfer entfernt. Abschließend wurde das Rohprodukt an Kieselgel chromatographiert (Laufmittel Petrolether:Essigester 8:2). Ausbeute: 201 mg eines farblosen Öls (64 % d.Th.).

### Beispiel 4

### N-t-Butoxycarbonyl-(R)-Cyclohexylglycinyl-(R,S)-dehydroprolinmethylester

Zu einer auf 0°C gekühlten Lösung von N-t-Butoxycarbonyl-(R)-Cyclohexylglycinylpyrrolin (524 mg, 1,70 mmol) und Dimethylcarbonat (0,3 ml, 357 mmol) in 5 ml trockenem THF wurde eine LDA-Lösung (2M, 2,6 mmol - Fluka) zugetropft. Nach 15minütigem Rühren bei 0°C wurde das Reaktionsgemisch auf 1N HCl/n-Pentan gegeben, mit NaHCO₃-Lsg. und Brine gewaschen und anschließend über MgSO₄ getrocknet. Nach Entfernen der Lösungsmittel am Rotationsverdampfer wurden 0,59 g eines gelben Öls enthalten. Die abschließende Reinigung erfolgte durch Chromatographie an Kieselgel mit PE:EE 7:3 als Eluens. Weißer, klebriger Feststoff. Ausbeute: 307 mg (49 % d.Th.).

## Patentansprüche

1. Verfahren zur Herstellung von 3,4-Dehydroprolinen und 3,4-Dehydropiperidinen der allgemeinen Formel I 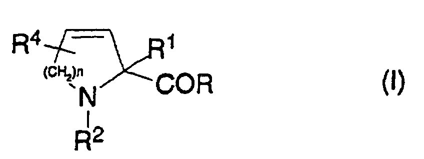 wobei
R Wasserstoff, C₁-C₆-Alkoxy, -NHC₁-C₆-Alkyl, -N(C₁-C₆-Alkyl)₂, OH, NH₂
R¹ Wasserstoff, C₁-C₆-Alkyl, Me₃Si, C₁-C₆-Alkyl-S
R² Boc, C₁-C₆-Acyl, Mesyl, Benzolsulfonyl, Tosyl, Trifluoracetyl, A1-A3-Peptid
n 1, 2
R⁴ H, C₁-C₆-Alkylrest
bedeuten,
enthaltend die Schritte Umsetzung eines Pyrrolin- oder Dehydropiperidinderivats der allgemeinen Formel II 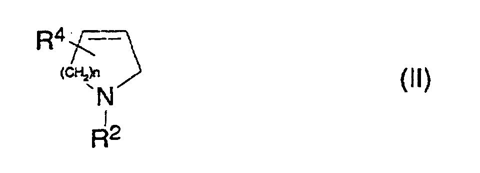 mit einer starken Base und einer Verbindung der allgemeinen Formel III 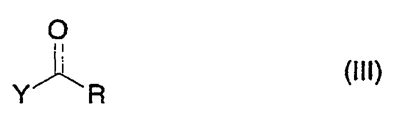 wobei
Y Cl, C₁-C₆-Alkoxy, -NHC₁-C₆-Alkyl, -N(C₁-C₆-Alkyl)₂, N(C₁-C₆-Alkyl)OC₁-C₆, wobei R nicht OH ist, bedeutet
oder mit Kohlendioxid für R = OH
und das Umsetzungsprodukt aus II und III hydrolysiert wird oder mit einer Verbindung der allgemeinen Formel IV
R³― X (IV)
wobei
X Cl, Br, I, MesO, TosO, Triflat
R³ Wasserstoff, C₁-C₆-Alkyl, Me₃Si, C₁-C₆-Alkyl-S oder NH₄ oder
R³-X gleich (C₁-C₆-Alkyl-S)₂ bedeuten,
umgesetzt wird.

2. Verfahren nach Anspruch 1, wobei die starke Base ein Alkalimetallamid ist.

3. Verfahren nach einem der Ansprüche 1 oder 2, wobei die Verbindung III Di-C₁-C₆-Alkylcarbonat ist.

4. Verfahren nach einem der vorherigen Ansprüche, **dadurch gekennzeichnet, daß** A1-A3-Peptid für alle enantiomeren bzw. diastereomeren Formen steht.

5. Verfahren nach einem der vorherigen Ansprüche, **dadurch gekennzeichnet, daß** im Fall chiraler Reste R² ein Diastereomer im Überschuß gebildet werden kann.

6. Verfahren nach einem der vorherigen Ansprüche, **dadurch gekennzeichnet, daß** man die Reaktion im Druckbereich von 1 bis 200 bar und einer Reaktionstemperatur zwischen -100 und +100°C durchführt.

## Claims

1. A process for the production of 3,4-dehydroprolines and 3,4-dehydropiperidines of the formula I 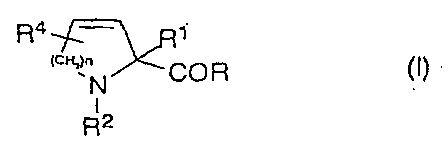 wherein
R means hydrogen, C₁-C₆ alkoxy, -NHC₁-C₆-alkyl, -N(C₁-C₆-alkyl)₂, OH, NH₂
R¹ means hydrogen, C₁-C₆-alkyl, Me₃Si, C₁-C₆-alkyl-S
R² means Boc, C₁-C₆-acyl, mesyl, benzenesulfonyl, tosyl, trifluoroacetyl, A1-A3 peptide
n means 1, 2
R⁴ means H, C₁-C₆ alkyl residue,
comprising the steps of reacting a pyrroline or dehydropiperidine derivative of the formula II 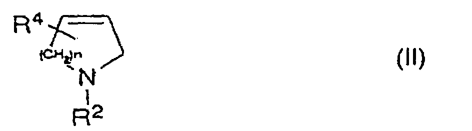 with a strong base and a compound of the formula III 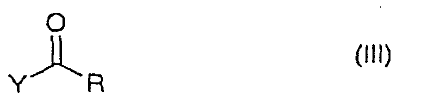 wherein
Y means Cl, C₁-C₆-alkoxy, -NHC₁-C₆-alkyl, -N(C₁-C₆-alkyl)₂, N(C₁-C₆-alkyl)OC₁-C₆, wherein R is not OH,
or with carbon dioxide where R = OH
and the reaction product of II and III is hydrolysed or is reacted with a compound of the formula IV
R³― X (IV)
wherein
X means Cl, Br, I, MesO, TosO, triflate
R³ means hydrogen, C₁-C₆-alkyl, Me₃Si, C₁-C₆-alkyl-S or NH₄ or
R³-X means (C₁-C₆-alkyl-S)₂.

2. A process according to claim 1, wherein the strong base is an alkali metal amide.

3. A process according to one of claims 1 and 2, wherein compound III is di-C₁-C₆-alkyl carbonate.

4. A process according to any one of the preceding claims, **characterised in that** A1-A3 peptide denotes all enantiomeric or diastereomeric forms.

5. A process according to any one of the preceding claims, **characterised in that**, in the case of chiral residues R², one diastereomer may be formed in excess.

6. A process according to any one of the preceding claims, **characterised in that** the reaction is performed in a pressure range of from 1 to 200 bar and at a reaction temperature of between -100 and +100°C.

## Revendications

1. Procédé de préparation de 3,4-déshydroprolines et de 3,4-déshydropipéridines de formule générale I 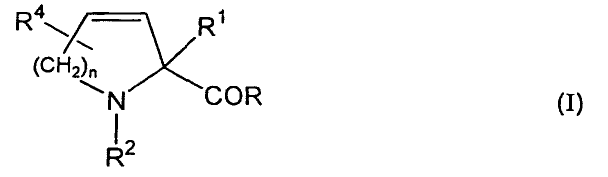 dans laquelle
R signifie l'hydrogène, un radical alcoxy en C₁-C₆, -NH-alkyle en C₁-C₆, - N(alkyl en C₁-C₆)₂, OH, NH₂,
R¹ signifie l'hydrogène, un radical alkyle en C₁-C₆, Me₃Si, alkyl en C₁-C₆-S,
R² signifie un radical BOC, acyle en C₁-C₆, mésyle, benzènesulfonyle, tosyle, trifuoracétyle, A1-A3-peptide,
n signifie 1, 2,
R⁴ signifie H, un radical alkyle en C₁-C₆,
comprenant les étapes consistant à faire réagir un dérivé de pyrroline ou de déhydropipéridine de formule générale II 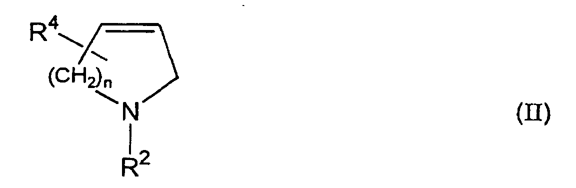 avec une base forte et un composé de formule générale III 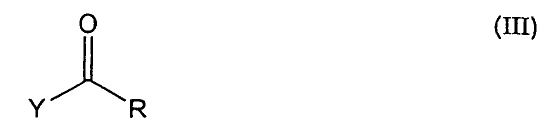 dans laquelle
Y signifie Cl, un radical alcoxy en C₁-C₆, -NH-alkyle en C₁-C₆, -N(alkyl en C₁-C₆)₂, N(alkyl en C₁-C₆)OC₁-C₆, où R n'est pas OH,
ou avec le dioxyde de carbone pour R = OH
et hydrolyser le produit de réaction de II et III ou le faire réagir avec un composé de formule générale IV
R³―X (IV)
dans laquelle
X signifie Cl, Br, I, un radical MesO, TosO, triflate
R³ signifie l'hydrogène, un radical alkyle en C₁-C₆, Me₃Si, alkyl en C₁-C₆-S ou NH₄
ou
R³-X est identique à (alkyl en C₁-C₆-S)₂.

2. Procédé selon la revendication 1, dans lequel la base forte est un amide de métal alcalin.

3. Procédé selon l'une des revendications 1 ou 2, dans lequel le composé III est le dialkyl(en C₁-C₆)carbonate.

4. Procédé selon l'une des revendications précédentes, **caractérisé en ce que** le A1-A3-peptide représente toutes les formes énantiomères, respectivement diastéréoisomères.

5. Procédé selon l'une des revendications précédentes, **caractérisé en ce que** dans le cas de radicaux chiraux R² on peut obtenir un diastéréoisomère en excès.

6. Procédé selon l'une des revendications précédentes, **caractérisé en ce qu'**on effectue la réaction dans une plage de pression de 1 à 200 bar et à une température réactionnelle entre - 100 et + 100 °C.
